# EUROPEAN PATENT APPLICATION

(11) **EP 4 763 156 A1**
(43) Date of publication of application: **24.06.2026**
(21) Application number: 24221211.6
(22) Date of filing: 18.12.2024
(51) Int. Cl.: A61F 9/007

(54) **VITREOUS SURGICAL PROBE**

(71) Applicant: Mani, Inc., Tochigi 321-3231 (JP)
(72) Inventor: KAWAMURA, Takanori, Utsunomiya-shi, 321-3231 (JP); YAMAGISHI, Konatsu, Utsunomiya-shi, 321-3231 (JP); TAZAWA, Yoshiyuki, Utsunomiya-shi, 321-3231 (JP)
(74) Representative: Gulde & Partner

(57) **Abstract**

A vitreous surgical probe 100 includes: a probe body 110 penetrating an eyeball; and a cutter blade 120 sliding along the inner surface of the probe body 110, in which the probe body 110 includes: a tubular portion 111 with an opening 111a in vicinity of a tip portion thereof; and a tip wall portion 112 sealing the tip portion of the tubular portion 111, the tip wall portion 112 is inclined such that a part located on the opposite side of the opening 111a is toward a proximal side, and, in a second cross-sectional shape on a second plane passing through the central axis of the tubular portion 111 and perpendicular to the first plane extending from a side of the opening to the opposite side of the opening, at least outer edges a of the tip wall portion 112 are positioned on the proximal side.

## Description

### BACKGRAOUND OF THE INVENTION

### Field of the Invention

The present invention relates to a vitreous surgical probe used in ophthalmic surgery.

### Description of the Related Art

Vitreous surgical probes used in ophthalmic surgery are employed to draw in and cut or remove (resect) jelly-like vitreous inside the eyeball and a proliferative membrane on a retina due to degeneration of vitreous (referred to as "vitreous and pathological vitreous"), through an opening of a probe body. When the vitreous and/or the pathological vitreous is near or floating around the retina, it is desirable to bring the probe body close to the retina.

Japanese Unexamined Patent Publication No. 2014-42703, filed by the present applicant, discloses a vitreous surgical probe that makes it easier to bring the probe body close to the retina by making the tip of the probe with an inclined surface.

The contents of Japanese Unexamined Patent Publication No. 2014-42703 are incorporated herein by reference in their entirety.

### SUMMARY OF INVENTION

### Problems to be Solved by the Invention

The more easily the opening of the probe body of the vitreous surgical probe as described above is placed close to the rentina, the easier it is to resect the vitreous and/or pathological vitreous near the retina.

The present invention has been made in view of the above point and it is an object of the present invention to make it easier to bring the opening of the probe body closer to the retina.

### Means of Solving the Problem

[1] To achieve the above object, the present technology discloses a vitreous surgical probe includes: a probe body penetrating an eyeball; and a cutter blade with an outer diameter fitting an inner diameter of the probe body, sliding along the inner surface of the probe body,
   in which the probe body includes: a tubular portion with an opening in vicinity of a tip portion thereof; and a tip wall portion sealing the tip portion of the tubular portion,
   the cutter blade includes a cutting edge at a tip thereof,
   the tip wall portion is inclined so that, in a first cross-sectional shape on a first plane passing through a central axis of the tubular portion and extending from a side of the opening to an opposite side of the opening, a part located on the opposite side of the opening at a tangent line passing through an intersection of the central axis is toward a proximal side, and a part from the opposite side of the opening to the intersection of the central axis in the first cross-sectional shape is positioned on the tangent line or proximally relative to the tangent line, and in a second cross-sectional shape on a second plane passing through the central axis of the tubular portion and perpendicular to the first plane, at least outer edge portions of the tip wall portion are positioned proximally relative to the intersection of the central axis.
   The invention allows the distance from the retina and similar structures to the opening to be set relatively short by tiling the probe body so that the tip wall portion is in close proximity to and aligned with the retina and similar structures. Furthermore, by rotating the probe body around the central axis of the tubular portion, the distance from the retina and similar structures to the opening can be further shortened.
[2] In the vitreous surgical probe according to [1], in which the second cross-sectional shape of the tip wall portion may be defined by a curved portion having a curved convex shape throughout its length.
[3] In the vitreous surgical probe according to [1], in which the tip wall portion may be designed such that the first cross-sectional shape thereof is straight, and the second cross-sectional shape thereof may include a pair of inclined flat portions sloping proximally on both sides of a ridge defined by a straight portion corresponding to the first cross-sectional shape of the tip wall portion.
[4] In the vitreous surgical probe according to [1], in which the tip wall portion may be designed such that the first cross-sectional shape thereof is straight, and the second cross-sectional shape thereof may include: a straight portion corresponding to the first cross-sectional shape of the tip wall portion; a central flat portion with a pair of edges on both sides of the traight portion; and a pair of inclined flat portions connected to the pair of edges of the central flat portion and sloping proximally toward both sides of the central flat portion.
[5] In the vitreous surgical probe according to [1], wherein the tip wall portion may include a symmetrical shape with respect to the first plane.

### Effects of the Invention

According to the present invention, it is possible to make the opening of the probe body easier to bring closer to the retina.

### BRIEF DESCRIPTION OF THE DRAWINGS

FIG. 1 is an explanatory view showing the usage state of a vitreous surgical probe;
FIG. 2 is a front view showing the state where a tip portion of a probe body is brought closer to a retina;
FIG. 3 shows a side view showing the state where the tip portion of the probe body is brought closer to the retina;
FIG. 4 shows a perspective view showing the structure of the tip portion of the probe body;
FIG. 5 shows a cross-sectional view of the tip portion of the probe body in the first plane P1;
FIG. 6 shows a cross-sectional view of the tip portion of the probe body in the second plane P2;
FIG. 7 shows a side view showing the state where an opening of the probe body is brought closer to the retina;
FIG. 8 shows a cross-sectional view of a tip portion of the probe body in the second plane P2 of an embodiment 2; and

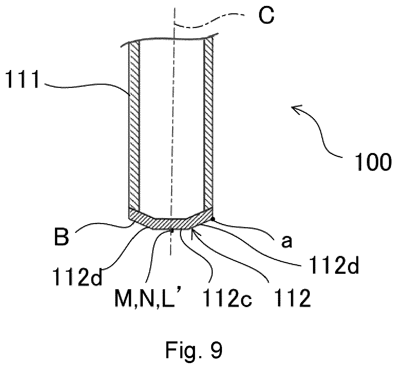

FIG. 9 shows a cross-sectional view of a tip portion of the probe body in the second plane P2 of an embodiment 3.

### DESCRIPTION OF THE EMBODIMENTS

Hereinafter, embodiments of the present invention are described in detail based on the drawings. In the following embodiments and modifications, components having the same functions as those in other embodiments are denoted by the same reference numerals, and their explanations are omitted. Furthermore, the components of each embodiment may be variously combined as logically possible.

### Embodiment 1

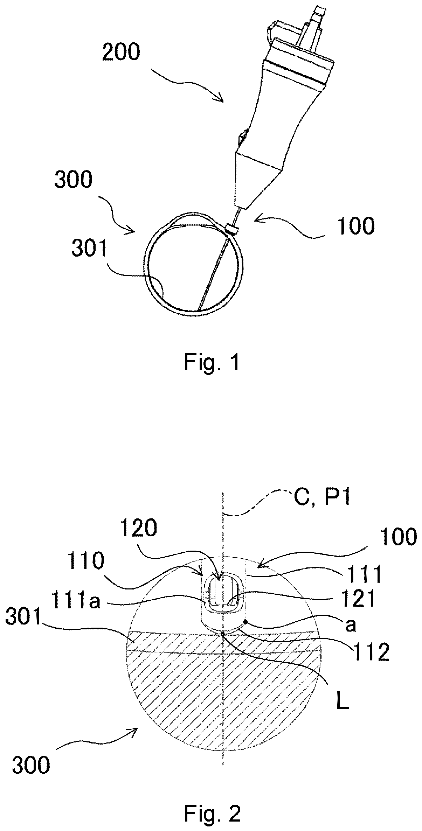

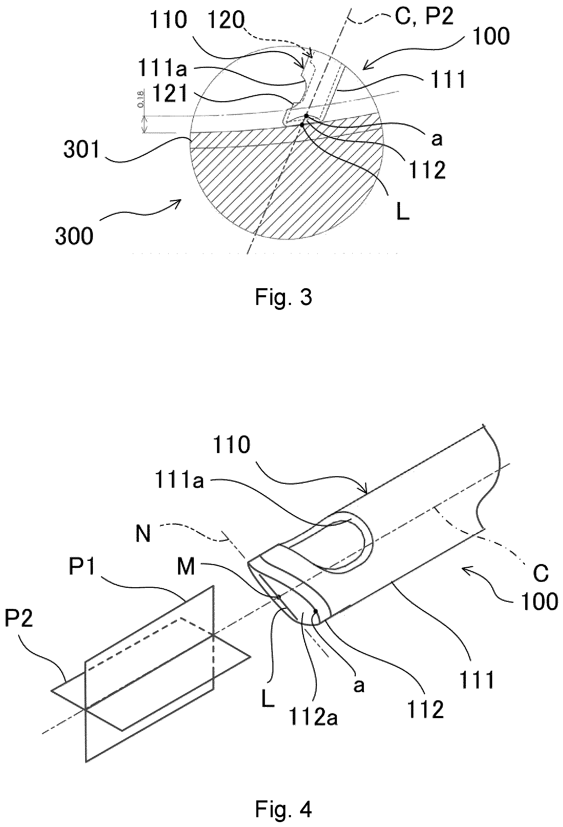

As shown in FIG. 1, a vitreous surgical probe 100 is connected to a vitreous cutter 200, penetrating an eyeball 300 to cut, remove (resect), and collect vitreous and/or pathological vitreous near a retina 301. The vitreous surgical probe 100 includes a probe body 110 and a cutter blade 120, as shown in FIGs 2 and 3.

The probe body 110 includes a cylindrical (e.g., tubular and pipe-like) tubular portion 111. A tip porion of the tubular portion 111 is sealed by a tip wall portion 112. This probe body 110 is an outer tube covering the cutter blade 120.

At the vicinity of the tip of the tubular portion 111 of the probe body 110, an opening 111a, which may be circular, is provided. This opening 111a is a hole (aperture) for attracting intraocular tissue such as the vitreous and/or pathological vitreous into the probe body 110 by suction from a base end side of the probe body 110 and cutting and collecting the intraocular tissue by sliding the cutter blade 120. The cut pieces of intraocular tissue such as the vitreous and/or pathological vitreous are, for example, suctioned and collected toward the base end side of the probe body 110.

FIG. 3 shows a side profile of the tip wall portion 112. The tip wall portion 112 of the probe body 110 is inclined such that an edge located on a side of the opening 111a of the tubular portion 111 constitutes a starting point of a top portion L, and an edge located on the opposite side of the opening 111a constitutes an endpoint of the top portion L, with the edge that serves as the starting point of the top portion L being positioned more distally relative to the edge that serves as the endpoint (first inclination). In addition, a surface of the tip wall portion 112 has a convex shape (shape including second inclination shape), maintaining a bulge on both sides of the top portion L as a ridge while gradually inclining more toward a proximal side of the probe body 110.

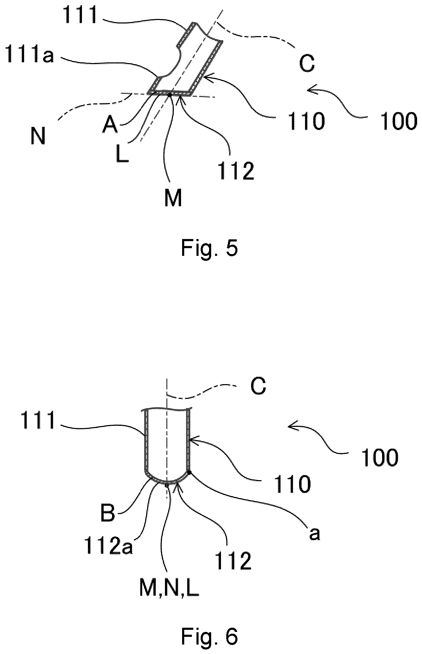

The tip wall portion 112 is described in more detail in FIGs. 4 to 6. The tip wall portion 112 is inclined such that, in the first cross-sectional shape A (FIG. 5) on the first plane P1 (FIG. 4) passing through the central axis C of the tubular portion 111 and extending from the side of the opening 111a to the opposite side of thereof, a part located on the opposite side of the opening 111a at a tangent line N (which coincides with the top portion L in this embodiment since the top portion L is straight) passing through an intersection M where the tip wall portion 112 intersects with the central axis C is toward the proximal side of the probe body 110, and a part from the opposite side of the opening 111a to the intersection M of the central axis C in the first cross-sectional shape A is positioned on the tangent line N (the part may have a curved shape so as to be positioned proximally relative to the tangent N). Furthermore, in the second cross-sectional shape B (FIG. 6) on the second plane P2 (FIG. 4) passing through the central axis C of the tubular portion 111 and perpendicular to the first plane P1, at least outer edge portions a of the tip wall portion 112 (the outer edge portions of the tip wall portion 112 are positioned at 90 degrees and 270 degrees, respectively, in projection onto a plane perpendicular to the central axis C when rotation phases of the starting point and endpoint of the top portion L are 0 degrees and 180 degrees) is defined by a curved portion 112a having a curved convex shape throughout its length that is positioned on the proximal side of the probe body 110 relative to the intersection M with the central axis C.

In the example of FIG. 6, in the second cross-sectional shape B of the tip wall portion 112 in the second plane P2 (FIG. 4), each area of the curved portion 112a on both sides of the top portion L are positioned on the proximal side of the probe body 110 relative to the top portion L. The same applies to the cross-sectional shape of the tip wall portion 112 in other planes parallel to the second plane P2.

The method of forming the tip wall portion 112 is not particularly limited, and for example, the tip wall portion 112 may be formed by welding it to the tubular portion 111 or by drawing a tip of the tubular portion 111 to form the shape of the tip wall portion 112.

The cutter blade 120 is for cutting the vitreous and/or pathological vitreous. The cutter blade 120 has a cylindrical (e.g., tubular and pipe-like) appearance similar to the tubular portion 111 of the probe body 110 and is provided with a cutting edge 121 at a tip thereof. The cutter blade 120 corresponds to an inner tube housed within the probe body 110, functioning as the outer tube. The cutter blade 120 is provided so as to slide along an inner surface of the probe body 110 in the axial direction of the probe body 110. Therefore, the cutter blade 120 has an outer diameter specifically designed to fit an inner diameter of the probe body 110. This allows the vitreous and/or pathological vitreous near the opening 111a to be cut into smaller pieces when the cutter blade 120 slides (advances and retreats) along the inner surface of the probe body 110, and the cutting edge 121 at the tip of the cutter blade 120 passes through the opening 111a (the other cutting edge).

As shown in FIG. 5, a part of the tip wall portion 112 on the side of the opening 111a in the vitreous surgical probe 100 configured as described above is inclined toward a tip side thereof (first inclination). This allows the distance from the retina 301 to the closest position on the periphery of the opening 111a to be set relatively short, for example, 0.18 mm, by tilting the probe body 110 with such a tip wall portion 112 so that the tip wall portion 112 is in close proximity to and aligned with the retina 301, as shown in FIG. 3.

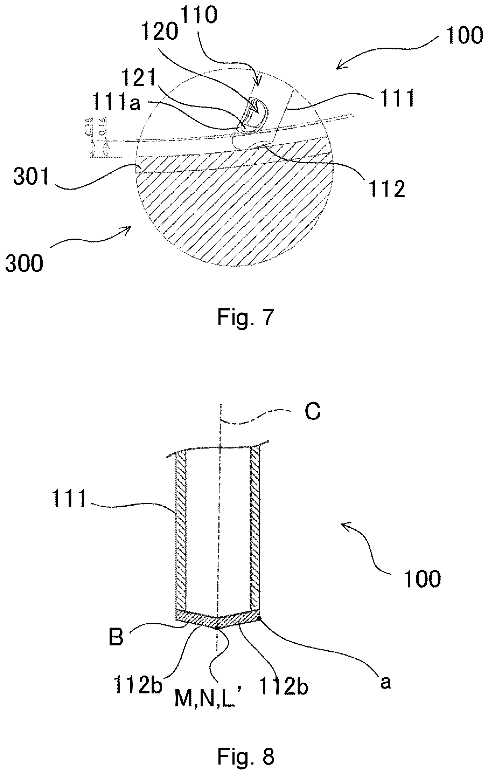

Furthermore, in the vitreous surgical probe 100 of this embodiment, the surface of the tip wall portion 112 has the convex shape (shape including second inclination shape), maintaining the bulge on both sides of the top portion L as a ridge while gradually inclining more toward the proximal side of the probe body 110, as shown in FIG.6. This allows the distance from the retina 301 to the periphery of the opening 111a to be further shortened, for example, 0.16 mm, by rotating the probe body 110 around the central axis C from the state shown in FIG. 3 to the state shown in FIG. 7.

More specifically, as shown in FIG. 3, when the probe body 110 tilted with respect to the retina 301 is rotated around the central axis C, the amount of movement toward the retina 301 at each part of the tip wall portion 112 is maximized at the outer edge portions a of the tip wall portion 112 (the outer edge portions of the tip wall portion 112 positioned at 90 degrees and 270 degrees in the projection onto the plane perpendicular to the central axis C when the rotation phases of the starting point and endpoint of the top portion L are 0 degrees and 180 degrees) in the second cross-sectional shape B (FIG. 6). However, since at least the outer edge portions a are positioned on the proximal side of the probe body 110 relative to the position along the central axis C of the intersection M where the tip wall portion 112 intersects with the central axis C, the probe body 110 can be rotated around the central axis C without contacting the retina 301 with the probe body 110 and without moving the probe body 110 much in the direction along the central axis C. This makes it easier to bring the opening 111a closer to the retina 301, facilitating the resection of the vitreous near the retina 301. In other words, by forming the inclined surface at the tip of the probe body to have both the first and second inclinations, it becomes easier to bring the opening 111a, which serves as an inlet for the vitreous and/or pathological vitreous, closer to the retina, allowing the opening 111a to be brought as close as possible to the vitreous and/or pathological vitreous to be collected, thereby reducing the possibilities of leaving the vitreous and/or pathological vitreous behind. In the above example, it is described, for convenience of explanation, that the probe body 110 in the state shown in FIG 3 is rotated around the central axis C to reach the state shown in FIG 7, but it is not limited to operating in two stages; for example, the probe body 110 may be designed to reach the state shown in FIG 7 at the time of insertion into the eyeball 300.

Here, the significance of bringing the opening 111a closer to the retina 301 is not limited to shortening the distance to the closest position on the periphery of the opening 111a from the retina 301; if an opening portion that effectively acts for the resection and suction of the vitreous is brought substantially closer to the retina 301, it will become easier to perform the resection of the vitreous near the retina 301.

The shapes of the opening 111a and the tip wall portion 112 can be designed symmetrically with respect to a plane including the central axis C of the tubular portion 111, allowing the distance between the opening 111a and the retina 301 to be shortened regardless of the inclination direction or rotation direction of the vitreous surgical probe 100, but it is not limited to this; it may also be designed in an asymmetric shape.

In FIgs 2 to 5, for convenience, the probe body 110 is depicted as being in close contact with the retina 301, but in actual use, it is recommended to keep the probe body 110 slightly elevated without contacting the retina 301 to avoid damaging it. Even in such a case, the effect of reducing the distance to the periphery of the opening 111a is fundamentally the same.

### Embodiment 2

A shape in which the outer edge portions a in the second cross-sectional shape B of the tip wall portion 112 are positioned on the proximal side of the probe body 110 relative to the position along the central axis C of the intersection M where the tip wall portion 112 intersects with the central axis C is not limited to the curved convex shape of the curved portion 112a as shown in FIG. 6; it may be designed such that the first cross-sectional shape A in the first plane P1 (FIG. 4) is straight as shown in FIG. 5, and the second cross-sectional shape B in the second plane P2 (FIG. 4) has a pair of inclined flat portions 112b sloping proximally on both sides of a ridge defined by a straight portion L' shown in FIG. 8.

In the example of FIG 8, in the second cross-sectional shape B of the tip wall portion 112 in the second plane P2 (FIG 4), each region of the inclined flat portions 112b on both sides of the straight portion L' is positioned on the proximal side of the probe body 110 relative to the straight portion L'. The same applies to the cross-sectional shape of the tip wall portion 112 in other planes parallel to the second plane P2.

### Embodiment 3

Furthermore, a shape in which the outer edge portions a in the second cross-sectional shape B of the tip wall portion 112 are positioned on the proximal side of the probe body 110 relative to the position along the central axis C of the intersection M where the tip wall portion 112 intersects with the central axis C may be designed such that the first cross-sectional shape A is straight as shown in FIG. 5, and the second cross-sectional shape B has a straight portion L', a central flat portion 112c with a pair of edges on both sides of the straight portion L', and a pair of inclined flat portions 112d connected to the pair of edges of the central flat portion 112c and sloping proximally toward both sides of the central flat portion 112c.
- 100: Vitreous surgical probe
- 110: Probe body
- 111: Tubular portion
- 111a: Opening
- 112: Tip wall portion
- 112a: Curved portion
- 112b: Inclined flat portion
- 112c: Central flat portion
- 112d: Inclined flat portion
- 120: Cutter blade
- 121: Cutting edge
- 200: Vitreous cutter
- 300: Eyeball
- 301: Retina
- P1: First plane
- P2: Second plane
- L: Top portion
- C: Central axis
- A: First cross-sectional shape
- B: Second cross-sectional shape
- M: Intersection
- N: Tangent line
- A: Outer edge
- L': Straight portion

## Claims

1. A vitreous surgical probe includes: a probe body penetrating an eyeball; and a cutter blade with an outer diameter fitting an inner diameter of the probe body, sliding along the inner surface of the probe body,
in which the probe body includes: a tubular portion with an opening in vicinity of a tip portion thereof; and a tip wall portion sealing the tip portion of the tubular portion,
the cutter blade includes a cutting edge at a tip thereof,
the tip wall portion is inclined so that, in a first cross-sectional shape on a first plane passing through a central axis of the tubular portion and extending from a side of the opening to an opposite side of the opening, a part located on the opposite side of the opening at a tangent line passing through an intersection of the central axis is toward a proximal side, and a part from the opposite side of the opening to the intersection of the central axis in the first cross-sectional shape is positioned on the tangent line proximally relative to the tangent line, and in a second cross-section shape on a second plane passing through the central axis of the tubular portion and perpendicular to the first plane, at least outer edge portions of the tip wall portion are positioned proximally relative to the intersection of the central axis.

2. The vitreous surgical probe according to claim 1, wherein the second cross-sectional shape of the tip wall portion is defined by a curved portion having a curved convex shape throughout its length.

3. The vitreous surgical probe according to claim 1, wherein the tip wall portion is designed such that the first cross-sectional shape thereof is straight, and the second cross-sectional shape thereof comprises a pair of inclined flat portions sloping proximally on both sides of a ridge defined by a straight portion corresponding to the first cross-sectional shape of the tip wall portion.

4. The vitreous surgical probe according to claim 1, wherein the tip wall portion is designed such that the first cross-sectional shape thereof is straight, and the second cross-sectional shape thereof comprises: a straight portion corresponding to the first cross-sectional shape of the tip wall portion; a central flat portion with a pair of edges on both sides of the traight portion; and a pair of inclined flat portions connected to the pair of edges of the central flat portion and sloping proximally toward both sides of the central flat portion.

5. The vitreous surgical probe according to claim 1, wherein the tip wall portion comprises a symmetrical shape with respect to the first plane.
